# EUROPEAN PATENT APPLICATION

(11) **EP 4 056 205 A1**
(43) Date of publication of application: **14.09.2022**
(21) Application number: 21162307.9
(22) Date of filing: 12.03.2021
(51) Int. Cl.: A61L 27/06, A61L 27/34, A61L 27/56

(54) **MICRO-RNA COMPOSITION FUNCTIONALIZED FOR DENTAL IMPLANTS**

(71) Applicant: Vilnius University Hospital Zalgiris Clinic, 08217 Vilnius (LT)
(72) Inventor: Puriene, Alina, 03104 Vilnius (LT); Jarmalaite, Sonata, 08101 Vilnius (LT); Butrimiene, Irena, 01124 Vilnius (LT); Punceviciene, Egle, 02222 Vilnius (LT); Snipaitiene, Kristina, 03101 Vilnius (LT); Rovas, Adomas, 06327 Vilnius (LT)
(74) Representative: Petniunaite, Jurga

(57) **Abstract**

The invention is directed to the medical field, and more particularly to a titanium dental implant functionalized with a selected combination of miR-140-3p, miR-145-5p, miR-146a-5p, and miR-195-5p inhibitors. Under the influence of miRNA inhibitors, the integration of the dental implant into the bone is accelerated, thus reducing the likelihood of dental implant failure and reducing the need for systemic antibacterial drugs. Also, due to more effective osseointegration, the dental implant can be used in patients with predisposed conditions - diabetes, osteoporosis, thyroid dysfunction.

## Description

### FIELD OF THE INVENTION

The invention relates to the field of medical technology, in particular, it is a titanium dental implant functionalized by coating with microRNA (miRNA) inhibitors in order to inhibit inflammation in the tissues around the implant and thus increase the likelihood of implant adhesion, reduce the time of implant integration into bone, and reduce the need for additional antibacterial systemic drugs.

### BACKGROUND OF THE INVENTION

Inflammation is a complex pathophysiological response of immune components to a deleterious factor associated with many diseases. During inflammation, pathogenic recognition receptors (PRRs) in cell membranes bind to molecules of pathogenic (bacterial lipopolysaccharides, outer membrane vesicles, fibrils, etc.) or endogenous origin (various intracellular proteins, amino acids, phospholipids, etc.) (1). Ligand interaction with PRR activates signal transduction pathways that regulate different transcriptional and post-transcriptional processes. The nuclear factor kappa light chain enhancer of activated B cells (NF-κB) belongs to a family of inducible transcription factors that are often referred to as major mediators of human inflammation (2). During inflammation, inactive NF-κB proteins in the cytoplasm detach from inhibitory proteins and, upon entry into the nucleus, bind to a specific region of DNA and promote transcription of gene targets. This in turn causes the production of inflammatory cytokines (IL-1, IL-2, IL-6, IL-8, IL-12, TNF-α), chemokines (MCP-1, IL-18, RANTES, MP-2, CXCL1, CXCL10), anti-apoptotic factors, and adhesion molecules (3). Increases in TNF-α, IL-1β, MCP-1, and other inflammatory cytokines and chemokines result in the migration of immune cells to the site of injury, differentiation of immune cells, and promotion of inflammation. Both inflammatory mediators, including NF-κB, inflammatory cytokines, chemokines, PRRs, and immune cells are continuously regulated by positive and negative feedback loops, and the amount of inflammatory mediators affects the homeostasis of the whole organism. Many diseases, especially autoimmune and chronic inflammatory diseases, are associated with inflammatory mediator imbalances and ineffective low-intensity chronic inflammation (4). Identification of an unbalanced inflammatory element and correction of its activity is one of the most effective strategies for the treatment of inflammatory diseases. For example, the treatment of autoimmune diseases (rheumatoid arthritis (RA), ankylosing spondylitis, etc.) is achieved with monoclonal antibodies that bind specifically to TNF-α and are effective in inhibiting inflammation (5). However, despite the positive treatment results, the widespread application of these drugs in daily practice is limited by the frequency of side effects and the extremely high production costs of the drugs (6).

One of the most promising therapeutic alternatives to currently used drugs is the epigenetic regulation of gene expression by miRNA. Epigenetics deals with inherited changes in gene function unrelated to changes in DNA sequence. One of the epigenetic regulatory mechanisms is miRNA. miRNA is a class of non-coding, single-stranded RNAs. Brandi miRNAs are on average 22 nucleotides in length and up to 10 nm³ in size. Approximately 2,500 mature human miRNAs are currently recorded in the databases, of which over 1,100 miRNAs have been validated (7). miRNA mainly negatively affects target genes by inhibiting the translation of specific informational RNAs and protein synthesis. miRNA regulates about 30% of the genes encoding proteins, thus contributing to many physiological and pathological processes (8). Deficiency or excess of miRNAs is associated with changes in the synthesis of the relevant proteins, which is why miRNAs are widely studied for the diagnosis of various conditions. Meanwhile, the use of mimic or antagonist miRNAs can adjust the concentration of endogenous miRNAs, thereby restoring altered synthesis, of target proteins. The extremely small size of the miRNA molecule, low systemic toxicity, small number of adverse reactions, low production costs and high potential to modulate the body's homeostasis are factors why miRNA is considered a highly promising therapeutic method for various cancers, autoimmune diseases and chronic inflammatory conditions.

One of the most common chronic inflammatory diseases is periodontitis (PD). It is an infectious disease characterized by inflammatory destruction of the connective tissue, resulting in loss of the periodontal junction and resorption of the alveolar bone. The incidence of PD can reach up to 70% of the population and increases in proportion to the age of the population - 70% of the population over the age of 65 have been found to have PD (9,10). PD is pathogenetically almost identical to inflammation around a dental implant called periimplantitis (11). The prevalence of periimplantitis is also particularly high, accounting for 10-20% of individuals with dental implants (12). In PD and periimplantitis, destruction of the surrounding tissues occurs due to inflammatory activation of osteoclasts. Osteoclasts make up 1-2% of bone cells. Osteoclasts are derived from hematopoietic myeloid cells, which also form monocytes, macrophages, and dendritic cells. Undifferentiated osteoclast precursors are found in bone marrow and blood. Differentiation of the mononuclear osteoclast precursor into the multinuclear active osteoclast is regulated by many systemic and local cytokines, from which two main factors are distinguished: M-CSF (macrophage colony-stimulating factor) and RANKL (receptor activator of nuclear factor kappa-B ligand). The main purpose of M-CSF is to increase the amount of osteoclast precursors, while the RANK ligand activates the NF-κB pathway and induces the binding of mononuclear osteoclast precursors to form multinucleated osteoclasts and induces the activation of osteoclast-specific genes, thereby stimulating functionally active osteoclasts.

It has been found that the intensity of inflammation in periodontal tissues and the differentiation of osteoclasts is significantly influenced by several dozen altered expression miRNAs (13). Several diagnostically and therapeutically promising miRNAs are: miR-146a, miR-140, miR-145, miR-218, miR-34a, miR-223, miR-142-3p, miR-148a. Lipopolysaccharides of periodontal pathogens bind to pathogen-recognizing receptors in the membrane and activate NF-κB, leading to miR-146a expression. miR-146a binds to membrane receptors and thus inhibits further NF-κB activation and inhibits inflammatory activation in this negative feedback (14). miR-146a inhibits the formation of reactive oxygen species, the expression of IL-1β, IL-6, IL-8, and TNF-α, and macrophage chemotaxis (15, 16). Decreased miR-146a expression is associated with pathological bone resorption (17). Experimentally elevated concentrations of miR-146a significantly reduce the inflammatory response in periodontal tissues (18). miR-146a deficiency is also associated with an increased risk of inflammatory and immune diseases such as RA, systemic lupus erythematosus, psoriasis, and osteoarthritis (19). Fushimi et al. (2018) found that miR-140 is involved in osteoblast differentiation processes by modulating Wnt3 and TGFβ3 signaling pathways (20). Meanwhile, miR-145 is known to inhibit IL-6 and IL-8 and to reduce IL-1β-induced membrane degradation (21, 22). Experimental increase in miR-145 inhibits macrophage differentiation, infiltration, and overall inflammatory intensity (23). miR-218 has been shown to inhibit TNF receptor and NF-κB signaling pathway activity and to negatively affect osteoclast differentiation and reduce bone resorption (24). miR-34a inhibits the expression of the osteoclast-specific growth factor Tgif2 (Transforming growth factor-beta-induced factor 2) and reduces osteoclast activation through inhibition of the RANKL signal (25). Several studies have found an increase in miR-223 expression in oral mucosal tissues in PD (26). miR-223 affects chemokine (CXCL2, CCL3) and interleukine (IL-6) quantity, and neutrophil infiltration into the site of chronic inflammation (27). Increases in miR-142-3p inhibit monocyte differentiation into macrophages and dendritic cell differentiation into osteoclasts. Thus, miR-142-3p inhibits the conversion of 3 major osteoclast precursors (monocytes, macrophages, and dendritic cells) to osteoclasts (28). miR-148a is significantly increased during M-CSF and RANKL-induced osteoclastogenesis. Administration of a miR-148a antagonist to mice with decreased bone density inhibited bone resorption and restored physiological bone density (29). Several *in vitro* studies are investigating the application of miRNAs to stimulate more efficient bone formation around dental implants (30-34). Various combinations of delivery systems and miRNAs have been used to functionalize titanium dental implants: titanium surface anodizing and bonding to miR-122 (30), miR-21 (31), miR-21 in combination with antimiR-138 (32), as well as surface coating with calcium ions and coupling to antimiR-138 (33) and coupling of gold nanoparticles and PLGA (polylactic-co-glycolic acid) to antimiR-204 (34). These *in vitro* studies used bone marrow and human mesenchymal stem cells and assessed changes in genes, miRNA and protein expression: alkaline phosphatase (ALP), bone morphogenic protein (BMP), collagen (COL1 and COL3), osteocalcin (OCN), osteopoetin (OPN) and others. Modified dental implants had no cytotoxic effect and induced osteogenic gene expression and bone mineralization (30-34). In most of these studies, the effect was assessed no later than 14 days after, possibly related to the degradation of miRNAs by enzymes (e.g. nucleases). Zhang, meanwhile, co-authored a methodology for surface modification of various biocompatible implants (dental implants, bone implants, cardiovascular stents) using chitosan gel coupling to a titanium implant via electrostatic interaction (CN104018199). Chitosan gel can be bound to various biological substances - siRNA, miRNA, DNA. Another method by the Zhang research team involves combining biological material (siRNA, miRNA) with biocompatible titanium implants through surface oxidation and encapsulation of miRNA in liposomes (CN102579145). Another patent application from a group of researchers from China describes the coupling of miRNA to dental implants using a hydrogel (CN107837243).

The articles and patent applications described herein do not specify how miRNAs are protected against nucleases, enzymes that degrade nucleic acids, including miRNAs. Endonucleases and exonucleases in the body are known to break down unprotected miRNAs and thus reduce their effectiveness. The methodology described in CN102579145, CN104018199 patent applications involves the coupling of siRNA with dental implants. siRNA is a double-stranded molecule of exogenous origin, in contrast to single-stranded endogenous miRNA. The use of siRNA can reduce the viability of surrounding cells by up to 40% (35). Also, said patent applications do not specify which miRNAs are used and do not disclose the intended effect. Meanwhile, patent application CN107837243 discloses the application of miR-21 to accelerate bone tissue calcification. The aforementioned articles describe the application of miR122, miR-21, miR-29b, antimiR-204, and antimiR-138 to improve implant osseointegration by inducing transcription of osteogenin genes (30-34).

### SUMMARY OF THE INVENTION

The present invention provides a titanium dental implant functionalized by coating with microRNA (miRNA) inhibitors. Anti-miRNA oligonucleotides inhibit proinflammatory miRNA activity therefore the level of inflammation in the tissues around the implant is reduced. This technology is unique, because in order to ensure slow and even delivery of unimpaired miRNA inhibitors to the cell multilayer liposomes are used. Our modified implants increase the likelihood of implant adhesion and reduce the time of implant integration into bone, ensuring successful implantation.

### BRIEF DESCRIPTION OF FIGURES

**Figure 1****.** Hierarchical clustering of differentially expressed miRNAs in periodontitis and control gingival tissue samples: miRNAs that were detected in ≥ 25% of the samples (A) and significantly deregulated miRNAs (N = 177, FC ≥ 1.5, P ≤ 0.050) (B).
**Figure 2****.** Relative expression of miR-140-3p, -145-5p, -146a-5p, and -195-5p in gingival tissues evaluated by means of RT-qPCR. Comparison of patients with periodontitis (PD-only), patients with both PD and RA (PD+RA+) and healthy controls (CT). The black lines denote median values, * P < 0.050, ** P < 0.010.
**Figure 3****.** Relative expression of miR-140-3p, -145-5p, -146a-5p, and -195-5p in gingival tissues collected from patients with severe periodontitis (PD, stage III and IV) and healthy controls (CT). The black lines denote median values, * P < 0.050, *** P < 0.001.
**Figure 4****.** Relative expression of miR-140-3p, -145-5p, -146a-5p, and -195-5p in gingival tissues collected from periodontitis patients and controls and compared according to cut-off points of mean clinical attachment loss (CAL) (A), periodontal probing depth (PPD) (B), bleeding on probing (BOP) (C) and bone loss (BL) (D). The black lines denote median values, * P < 0.050, ** P < 0.010 and *** P < 0.001.
**Figure 5****.** Fluorescence microscopy images of a vesicle containing NBD-Chol loading into a) TiO₂ nanotube arrays and b) polyethylenimine modified TiO₂ nanotube arrays.
**Figure 6****.** UV/Vis spectra of liposomes enclosed miRNA.
**Figure 7****.** Schematic plate layout of cell treatment variants. Different shades of grey represent different miRNR (miR) inhibitor concentrations used for transfection. NC - negative control.
**Figure 8****.** Schematic plate layout of cell treatment variants. I - titanium disks without pores and not associated with liposomes and/or miR inhibitors, II - perforated disks not associated with liposomes and/or miR inhibitors.

### DETAILED DESCRIPTION OF THE INVENTION

A combination of inflammation related PD, specific miRNAs were selected after analyzing miRNA expression profiles in PD-affected (with or without RA) and healthy gingival tissues.

### RNA extraction

Snap-frozen gingival specimens were ground to a fine powder. Total RNA was isolated using Trizol^{™} (Invitrogen, TFS, USA) reagent according to the manufacturer's protocol and stored at -80 °C.

For RNA extraction from cell culture samples miRNeasy Mini Kit (Qiagen) was used according to the manufacturer's instructions. After trypsinization and collection of the cells, QIAzol^{®} Lysis Reagent (Qiagen) was added to cell culture samples and incubated for 5 min. Following incubation, 200 µL of chloroform was added and incubated for additional 2-3 min. As a positive control for extraction efficiency, 25 fmol cel-miR-39 was used. Extracted RNA was stored at -80 °C. The quality and quantity of RNA was determined using NanoDrop 2000 (Thermo Scientific, USA) spectrophotometer and Qubit 4 (Invitrogen) fluorometer.

### MiRNA profiling by means of microarrays

A total of 16 samples were analyzed. Briefly, 100 ng of total RNA was spiked with control RNA Spike-In solution (Agilent Technologies (AT), USA), dephosphorylated, denatured and labeled with 3-pCp cyanine dye according to miRNA Microarray System with miRNA Complete Labeling and Hyb Kit (AT) protocol. After labeling, washed and dried samples were hybridized at 55 °C for 20 h on Human microRNA Microarray 8 x 60K arrays. Microarray images were acquired with Agilent SureScan scanner and analyzed using matching software (*Agilent Microarray Scan Control, Feature Extraction software v10.7, GeneSpring GX v14.9*) (AT, USA).

### cDNA synthesis

For the expression analysis of selected miRNAs in gingival tissue biopsies RT reaction was performed by following manufacturer's protocol using *TaqMan*^{®} *MicroRNA Reverse Transcription Kit* and specific *RT Primer Pool* mix (Applied Biosystems (ABI), TFS). After 12 µL/1 reaction of RT reaction mix was assembled, vortexed and centrifuged, 3 µL (350 ng) of total RNA was added and incubated on ice for 5 min. The RT reaction was performed in a thermocycler at 16°C for 30 min, 42 °C for 30 min, 85 °C for 5 min and was subsequently maintained at 4 °C.

Same *TaqMan*^{®} *MicroRNA Reverse Transcription Kit* was used for cDNA synthesis in cell culture samples, but for every gene and every sample separate cDNA reactions were performed, differing in miRNA-specific stem-loop primers (*TaqMan*^{®} *MicroRNA Assay* for miR-140-3p, -145, -146a, and -195; manufacturer's assay IDs - 002234, 002278, 000468 and 000494, respectively), following manufacturer's protocol (all from ABI, TFS) (Table 1). To 3.5 µL/1 reaction of reaction mix, 1.5 µL of miRNA assay and 2.5 µL (1-10 ng) of total RNA were added, centrifuged and then incubated in a thermocycler at the same conditions as described above. Synthesized cDNA was then either used immediately for the next step or stored at -20 °C.

**Table 1. Manufacturer's IDs and mature miRNA sequences of TaqMan^{®} Assays (Applied Biosystems, Thermo Fisher Scientific, USA).**

| **miRNA** | **ID** | **Mature miRNA Sequence** |
|---|---|---|
| miR-140-3p | 002234 | UACCACAGGGUAGAACCACGG |
| miR-145 | 002278 | GUCCAGUUUUCCCAGGAAUCCCU |
| miR-146a | 000468 | UGAGAACUGAAUUCCAUGGGUU |
| miR-195 | 000494 | UAGCAGCACAGAAAUAUUGGC |

### Quantitative Reverse Transcription Polymerase Chain Reaction (RT-qPCR)

Expression level of selected miRNAs samples were quantified in triplicates using custom *TaqMan*^{®} *Low Density Arrays* in gingival tissue samples from patients with PD and/or RA and healthy controls by following manufacturer's instructions (ABI, TFS). Reaction mix, consisting of 2 × *TaqMan Universal Master Mix II, No AmpErase UNG,* nuclease-free water and cDNA, was prepared for each sample separately. Array card was loaded by pipetting prepared reaction mix into the fill port of the channel. Cards were then centrifuged 2 times for 2 min at 2000 rpm, sealed and the fill reservoirs were trimmed off following the manufacturer's instructions. Array cards were run on the *ViiA7*^{™} *Real-Time PCR System* by using *ViiA* 7 *Software v1.2* (ABI, TFS) and the following temperature regime was applied: 2 min at 50 °C, 10 min at 95 °C, followed by 40 cycles of 15 s at 95 °C and 1 min at 60 °C.

miRNA abundance quantification in cell culture samples was performed by RT-qPCR, which final reaction volume (20 µL/1 reaction) consisted of 2× *TaqMan*^{™} *Universal Master Mix II, no UNG, 20*× *TaqMan*^{™} *MicroRNA Assay* (both from ABI, TFS), 1.33 µL cDNA and RNase-free water and by following manufacturer's protocol. Each sample was run in triplicates and no-template control (NTC) was performed for evaluation of contamination. In NTC, nuclease-free water was added instead of the cDNA sample. When all samples were pipetted into the appropriate wells, plate was sealed, briefly centrifuged to eliminate air bubbles and then loaded into the instrument. Reaction was performed on *ViiA7*^{™} *Real-Time PCR System* by using *ViiA* 7 *Software v1.2* and the following temperature regime was applied: 2 min at 50°C, 10 min at 95°C, followed by 50 cycles of 15 s at 95°C and 1 min at 60°C.

All data was analyzed using *SPSS* software, version 20.0 (IBM, Armonk, NY) and *GenEx* software, version 7.0 (MultiD Analyses AB, Sweden). miRNA data generated by RT-qPCR analysis was normalized using spiked-in cel-miR-39 and log-transformed (log2). Data was analyzed performing Student t-test and non-parametric tests. Associations were considered statistically significant when P ≤ 0.050 in all comparisons.

### Genome-wide miRNA expression profiling in gingival tissues

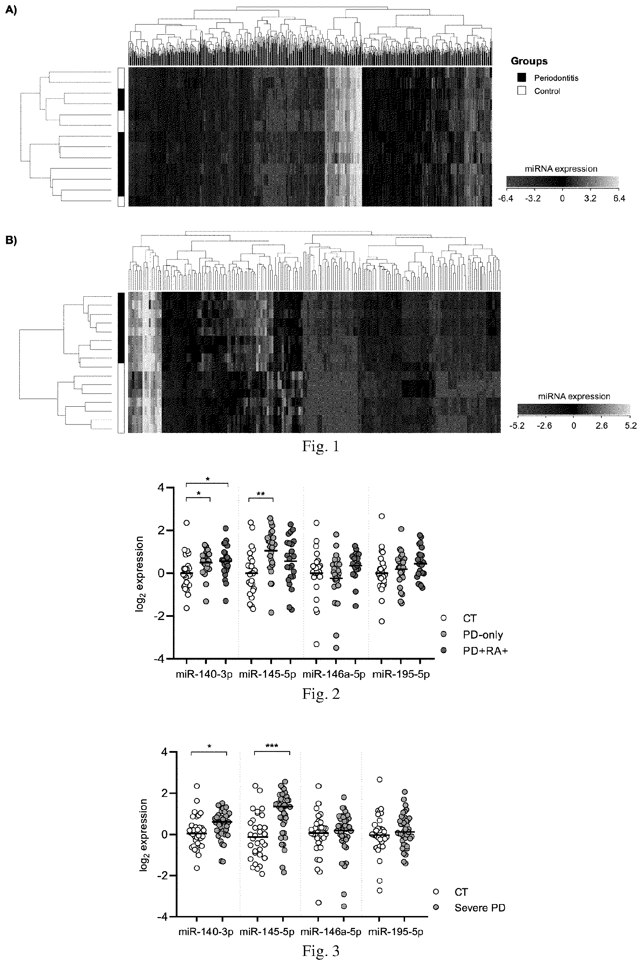

MiRNA expression profile was analyzed in 16 gingival tissue specimens: PD-affected (PD+) (N = 8) and PD-unaffected (PD-) (N = 8) tissues. Out of 2569 available miRNAs, 760 were detected in ≥ 25% of samples, and only these were left in further analysis (Fig. 1A).

MiRNA expression analysis in gingival samples by means of microarrays revealed 177 differentially expressed miRNAs in PD-affected tissues compared to periodontally healthy gingival tissues (PD+ vs. PD-) (Fig. 1B).
Majority of miRNAs (N = 140, 79.1%) were upregulated by >2-fold, including miR-30a-5p, -125a-3p, -126-5p, -1273g-3p, -140-3p, -145-5p, -146a-5p, -155-5p, -195-5p, -575, -630, and -3917. Specificity for PD was evaluated by comparing patients with PD-only (PD+RA-, N = 4) vs. healthy controls (CT, N = 4) as 118 significantly deregulated miRNAs were revealed. Majority of miRNAs (N = 77, 65.3%) were upregulated (miR-140-3p, -550a-3-5p, -765, -1273g-3p, -3917, etc.), while 41 (34.7%) miRNAs were downregulated (miR-1246, -210-3p, -375, etc.). To reveal which miRNAs may be associated with diseases of inflammatory nature, gingival samples from patients with PD and RA (PD+RA+, N = 4) vs. CT were compared. A general trend towards miRNA upregulation was observed as 22 out of 29 differentially expressed miRNAs were upregulated, including miR-550a-3-5p, -140-3p, and -765.

Results of miRNA microarray analysis were used to select miRNAs related to PD in patients with or without RA. The selection of these miRNAs was based on fold changes and P-values as well as their possible role in inflammation or autoimmune processes. MiRNAs showing the most significant upregulation in PD+ vs. PD-, PD-only vs. CT, and PD+RA+ vs. CT were selected. Nine miRNAs (miR-30a-5p, -125a-3p, -145-5p, -146a-5p, -155-5p, -195-5p, -423-5p, -575, and -630) were significantly overexpressed in PD+ vs. PD- and PD+RA+ vs. CT groups, while 4 miRNAs (miR-126-5p, -195-5p, -1273g-3p, and -3917¬) were upregulated in PD+ vs. PD- and PD-only vs. CT. Significant overexpression of miR-140-3p and -765 was observed in all three of aforementioned groups and in PD-only vs. CT, respectively. Furthermore, both miR-140-3p and - 765 were significantly upregulated in PD+RA+ vs. CT group.

Based on revealed associations, 15 miRNAs were selected for further analysis, namely: miR-30a-5p, -125a-3p, -126-5p, -140-3p, -145-5p, -146a-5p, -155-5p, -195-5p, -423-5p, -550a-3-5p, - 575, -630, -765, -1273g-3p, and -3917. In order to validate detected changes, selected miRNAs were further analysed by performing RT-qPCR in an extended cohort of gingival tissues (N = 80).

### Selected miRNA expression analysis in gingival tissue

RT-qPCR analysis of 80 gingival tissue samples was performed and it was confirmed that a part of selected miRNAs was significantly associated with periodontitis. As well as in genome-wide miRNA expression analysis, overexpression of miR-145-5p and miR-140-3p (FC = 2.1, P < 0.0001 and FC = 1.4, P = 0.019, respectively) was identified in gingival tissue specimens from patients with PD-only (N = 25) vs. CT (N = 25) (Fig. 2). Comparison of gingival tissue specimens from PD+RA+ group (N = 23) and CT revealed that miR-140-3p was upregulated (FC = 1.5, P = 0.017) (Fig. 3), as well as in microarray analysis. Meanwhile, the expression of miR-125a-3p and miR-1273g-3p was significantly lower (FC < -1.5; P < 0.05) in PD+RA+ vs. CT and PD+ vs. PD- (N = 32 vs. 48). Analysis of miR-146a expression in gingival tissue yielded no significant results.

The expression of gingival miR-145-5p and miR-140-3p increased significantly amongst participants with severe PD (Stage III and IV). Comparison of severe PD vs. CT revealed that expression of miR-145 and miR-140-3p increased significantly amongst participants with PD (FC = 2.2, P < 0.001 and FC = 1.3, P = 0.026, respectively) (Fig. 3), while expression of miR-125a-3p decreased by 2.2-fold (P < 0.001). Significant difference in miR-145-5p gingival level was observed by comparing severe PD with moderate PD (Stage II) (FC = 2.0, P = 0.007). Comparison of moderate PD vs. CT revealed significantly higher expression of miR-195-5p (FC = 1.7, P = 0.038) and lower expression of miR-125a-3p (FC = -3.5, P = 0.013).

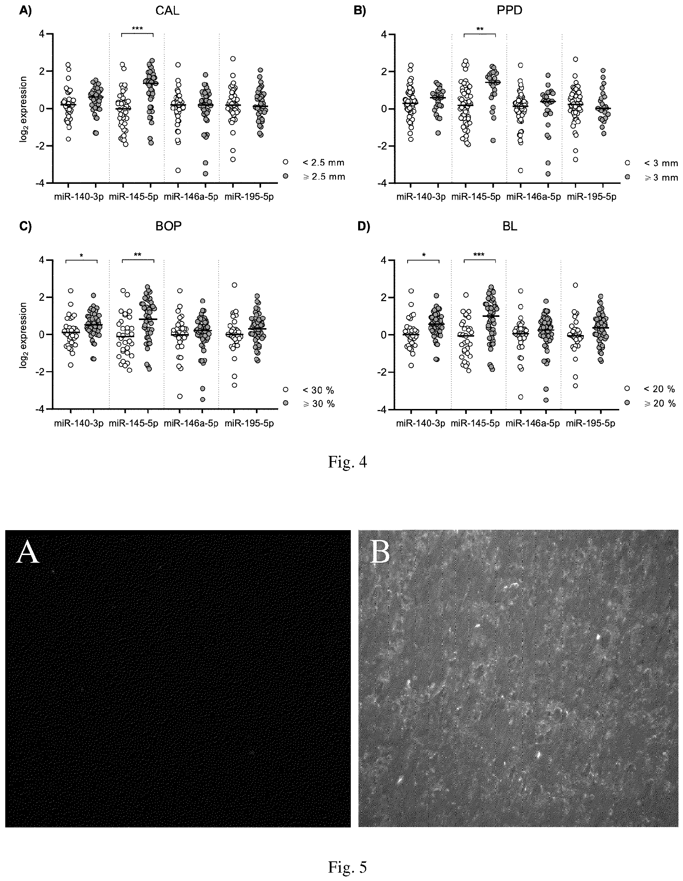

Significant associations of selected miRNAs were revealed with clinical-pathological parameters of PD, such as clinical attachment loss (CAL), periodontal probing depth (PPD), bleeding on probing (BOP) and bone loss (BL). In a group of patients with CAL score of ≥ 2.5 mm the expression of miR-145 was 2.2-fold higher (P < 0.001), while expression of miR-3917 - 1.6-fold lower (P = 0.022), as compared to the patients with CAL of < 2.5 mm (Fig. 4A). Upregulation of miR-145 (FC = 2.1, P < 0.001) in gingival tissue was also observed in participants with PPD of ≥ 3 mm as compared to the ones with PPD of < 3 mm (Fig. 4B). Overexpression of miR-145 (P < 0.001) and miR-140-3p (P = 0.009) as well as downregulation of miR-125a-3p by >2-fold (P = 0.001) was identified in patients with BOP of ≥ 33 % as compared to the ones with BOP of < 33 % (Fig. 4C). We also identified an upregulation of both miR-145 (P < 0.001) and miR-140-3p (P = 0.007) (Fig. 4D), as well as downregulation of miR-125a-3p (P = 0.001) when participants with BL of ≥ 20 % and BL of < 20 % were compared.

MiRNAs abundantly expressed in gingival tissues, namely, miR-140-3p, -145-5p, -146a-5p and -195-5p, were selected for further experiments. The selection was based on fold changes, P-values, correlation between their expression and clinical-pathological parameters of PD, as well as their possible role in inflammation or autoimmune processes. Inhibitors of selected miRNAs were used for its delivery to multilayer liposomes, *in vitro* and *in vivo* experiments.

### MiRNA inhibitors

We selected stem-loop type of inhibitors that are approx. 100 nt long and ensure the specific binding of mature miRNAs. As the same miRNA-specific stem-loop sequences are provided and have been used for both, transfection and reverse transcription steps of the study, the specificity and repeatability could be ensured. MiRNA inhibitors used in our study are commercialized (TFS) and therefore are tested and certified for use. Detailed information is provided in Table 2.

**Table 2. Details on sequences of selected miRNA and their stem-loops.**

| **miRNA, hsa-miR-** | **Inhibitor's assay ID** | **Mature miRNA sequence** | **Stem-loop sequence** | **Chromosome location,** on Build GRCh38 |
|---|---|---|---|---|
| -145-5p | MH11480 | GUCCAGUUU UCCCAGGAA UCCCU | CACCUUGUCCUCACGG UCCAGUUUUCCCAGGA AUCCCUUAGAUGCUAA GAUGGGGAUUCCUGGA AAUACUGUUCUUGAGG UCAUGGUU | Chr.5: 149430646 - 149430733 [+] |
| -140-3p | MH12503 | UACCACAGG GUAGAACCA CGG | UGUGUCUCUCUCUGUG UCCUGCCAGUGGUUUU ACCCUAUGGUAGGUUA CGUCAUGCUGUUCUAC CACAGGGUAGAACCAC GGACAGGAUACCGGGG CACC | Chr. 16 - 69933081 - 69933180 [+] |
| -195-5p | MH10827 | UAGCAGCAC AGAAAUAUU GGC | AGCUUCCCUGGCUCUA GCAGCACAGAAAUAUU GGCACAGGGAAGCGAG UCUGCCAAUAUUGGCU GUGCUGCUCCAGGCAG GGUGGUG | Chr. 17 - 7017615 - 7017701 [-] |
| -146a-5p | MH10722 | UGAGAACUG AAUUCCAUG GGUU | CCGAUGUGUAUCCUCA GCUUUGAGAACUGAAU UCCAUGGGUUGUGUCA GUGUCAGACCUCUGAA AUUCAGUUCUUCAGCU GGGAUAUCUCUGUCAU CGU | Chr. 5 - 160485352 - 160485450 [+] |

### MiRNA inhibitors delivery by surface coating technology

MiRNA delivery by the surface coating technology consists of two steps: the surface preparation of TiO₂ nanotube arrays and loading of vesicle-enclosed miRNA for sustainable and controllable release. TiO₂ nanotube arrays were prepared by self-ordering electrochemical anodization procedure by immersing Ti foil (99.9 at. % purity, 0.1 mm thick) in thermostated glass cell containing ethylene glycol with 0.3 wt. % NH₄F, 20 mL L⁻¹ H₂O at 20 ± 1 °C and applying 50 V for 30 min using two platinum plates as cathodes. After the procedure, the samples were baked in air at 450 °C for 2 hours. The procedure results in a uniform distribution of the TiO₂ nanotube array, where the diameter of the tube varies between 80 and 100 nm and the length along the tube is approx. 3 µm. Lastly, TiO₂ nanotube array were modified with poliethylenimine to capture the liposomes for controlled for sustainable and controllable release. To test the ability for liposome loading into TiO₂ nanotube array, fluorescence microscopy was employed. Unilamellar liposomes containing fluorescent agent - NBD-Chol ((22-(N-(7-Nitrobenz-2-Oxa-1,3-Diazol-4-yl)Amino)-23,24-Bisnor-5-Cholen-3β-Ol) were prepared using 10 nM chloroform solutions of DOPC (1,2-dioleoyl-sn-glycero-3-phosphocholine), Chol (Cholesterol), and 25 mM of NBD-Chol. A lipid film was formed by evaporating 60 µL DOPC, 40 µL Chol and 1 µL NBD-Chol of chloroform solutions in a stream of nitrogen for 1 h. After evaporation the lipid film was hydrated using buffer solution (0.1 M NaCl and 0.01 M NaH₂PO₄ at pH 7.4) with the vortex until the lipid film was no longer visible. The mixture was then extruder through a 30 nm polycarbonate membrane 21 times to yield a liposome size of approx. 25-35 nM (determined using dynamic light scattering). The liposomes containing fluorescent agent were incubated for 1 h into TiO₂ nanotube arrays. After the incubation, the TiO₂ nanotube arrays were washed with copious amount of buffer solution and deposited under fluorescence microscope. It is evident from the Fig. 5 image A that no fluorescence is detected of the NBD-Chol. However, polyethylenimine modified TiO₂ nanotube arrays exhibited highly fluorescent image, evidently from NBD-Chol containing liposomes (Fig. 5B).

### Liposome enclosed miRNA preparation

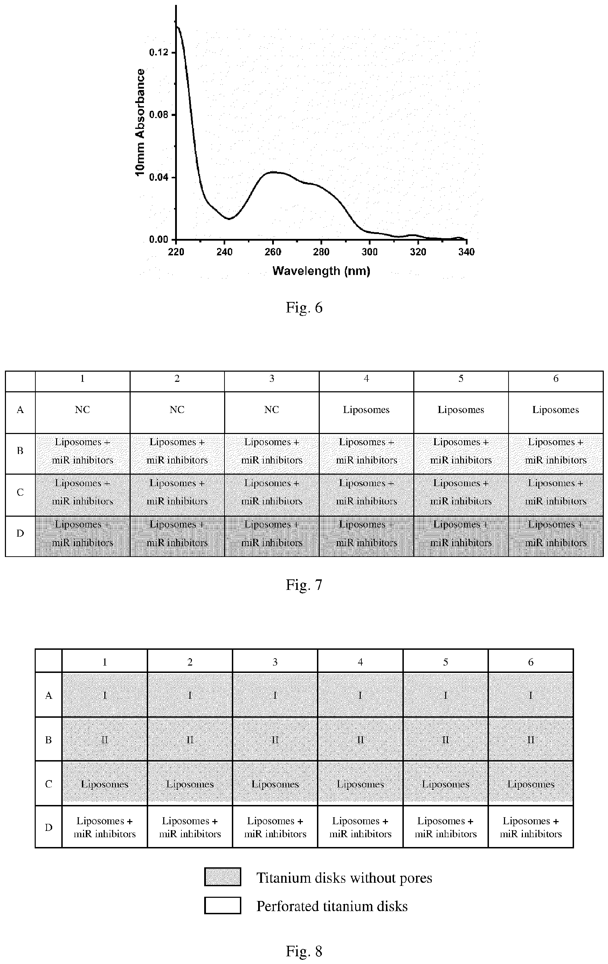

Liposome enclosed miRNA inhibitors was prepared using 10 nM chloroform solutions of DOPC, Chol and 1 µM miRNA aqueous solution. Firstly, a liposome suspension of 1.5 mM (molar % ratio of lipids was 60% DOPC and 40% cholesterol) was prepared by evaporating desired amount of DOPC and Chol lipid solution in a stream of nitrogen for 1 h. After evaporation, the lipid film was hydrated using buffer solution (0.1 M NaCl, 0.01 M NaH₂PO₄ and 1 µM miRNA inhibitor at pH 7.4) and with the vortex until the lipid film was no longer visible. The mixture was then extruded through a 100 nm polycarbonate membrane 21 times to yield a liposome size of approx. 100 nM. After extrusion, the liposome enclosed miRNA inhibitor solution was dialyzed using dialysis bag (molecular weight cut-off 12-14 kDA) submersed in copious amount of buffer solution (0.1 M NaCl, 0.01 M NaH₂PO₄ at pH 7.4) with a gently stirring for 48 hours at 4 °C. Dialysis procedure is necessary for removal of non-entrapped miRNA inhibitors. The volume difference between solutions (liposome solution and buffer solution) was approx. 1000 times. Buffer solution was also replaced after 24 hours. After dialysis, determination of inhibitor entrapment in liposomes was determined using UV/Vis spectroscopy, which can shows intrinsic absorptivity properties of nucleic acids (DNA and RNA), typically yielding a characteristic peak at 260 nm. After dialysis of liposomes, the solution was measured using UV/Vis spectroscopy (as a background scan, liposomes prepared by same procedure with the exception of miRNA inhibitor addition was used). As observed in Fig. 6, a typical absorption peak was observed at 260 nm, showing that nucleic acids, in this case, miRNA inhibitors are entrapped within the liposomes.

### Evaluation of the efficacy of biologically active liposomes in a cell culture model

To functionally prove that inhibitors of selected miRNAs, namely miR-140-3p, -145-5p, - 146a-5p and -195-5p, suppress function of corresponding miRNAs, by inhibiting their expression in cells, we performed miRNA expression analysis in differently treated cells grown on cell culture plate plastic. Mesenchymal stem cells, primarily induced by pro-inflammatory factors, were cultivated in 24-well plates at a density of 5 × 10³ cells/well and cultured in growth medium at 37 °C in a humidified atmosphere containing 5% CO₂. Then, cells were treated with 1) liposomes alone, 2) complex of liposomes and miRNA inhibitors, by the procedure called lipofection, or 3) left untreated and used as negative control (NC). Mix of all four selected miRNA inhibitors were used in three different concentrations in cells treated with both inhibitors alone and in a complex with liposomes. Experiment was performed in triplicates (Fig. 7). Transfection efficiency was assessed at three time points - 24, 48 and 72 hours after treatment.

RNA isolation from treated cells was followed by gene expression analysis by means of RT-qPCR. The expression of selected miRNAs between NC and cells treated with liposomes alone yielded no significant difference at all three time points after treatment. miRNA expression in cells treated with liposome and miRNA inhibitor complex remained practically the same in all observed time points. Generally, miRNA expression in cells treated with liposome and miRNA inhibitor complexes were significantly decreased as compared to NC or cells treated with liposomes alone. Results indicate that lipofection enables efficient anti-miR oligonucleotides to be delivered to the cell through its membrane where they inhibit expression of corresponding miRNAs.

As significant differences in levels of miRNA expression between the cells transfected with miRNA inhibitors and NC were found, we selected optimal concentration of inhibitors required to effectively suppress the expression of corresponding miRNAs and performed analogical experiment on titanium surfaces. Cells were cultivated in 24-well plate with bottom covered with titanium disks (Ø 1.9 cm²) of four kinds: 1) disks without pores, 2) perforated disks alone, 3) perforated disks associated with liposomes, and 4) perforated disks associated with liposomes containing miRNA inhibitors. Perforated disks that were not associated with liposomes and/or miR inhibitors were used as NC. Experiment was performed in 6 replicates (Fig. 8). Cell viability, changes in gene expression were assessed and release time of liposomes was evaluated at three time points - 24, 48 and 72 hours after treatment.

The percentage of cell viability and proliferation rate of the cells were similar in cells cultivated on both perforated titanium disks and disks without pores at all three time points after treatment. As well as in cells grown on plastic plates, expression of selected miRNAs between NC and cells grown on perforated disks that were associated with liposomes alone indicated no significant difference at all three time points after treatment. Stable suppression of selected microRNA expression in cells cultivated on perforated disks that were associated with liposomes containing miRNA inhibitors was observed at each time point. It indicates that liposomes are released slowly and evenly which is necessary to block proinflammatory miRNAs, ensure successful adhesion and prolong the usage of dental implants. Compared with cell-culture plate plastic, viability of cells growing on titanium surface was slightly lower, however, gene expression differences between aforementioned groups were analogical as in cells grown on plastic plates. It indicates that based on the physical properties of titanium and active cell proliferation on it, titanium appears to be suitable material for dental implants functionalized with a selected combination of miRNA inhibitors.

### Implantation of an anodized dental implant associated with biologically active liposomes in laboratory animals

Female rabbits aged 7-10 months old and weighing 3.5-4 kg were used for the study. Certain age was chosen taking into the account that the rabbit skeleton is fully developed by 6 months of age. 80% statistical power at 5% level significance the total sample size required for the study was 12. During the experimental period, the animals were kept under the same conditions of food and temperature.

Four kinds of anodized implants were used in the study: 1) implants without pores, 2) perforated implants alone, 3) perforated implants associated with liposomes, and 4) perforated implants associated with liposomes containing miRNA inhibitors.

Each rabbit received all four kinds of implants, two in each leg 7 mm apart from each other. Implants were placed in the prepared beds of proximal femur and threaded until the screw thread was completely introduced into the bone tissue.

In order to assess the bone formation process after implantation, euthanasia was performed at 3 different time points - 1, 3, and 6 weeks after the surgery, for 4 individuals at each time point. Therapeutic effect was evaluated by analyzing bone blocks with implants, assessing bone volume, trabecular number and density by means of micro-computed tomography.

One week after implant placement, there were signs of necrosis in external parts of the cortical bones, however bone neoformation was observed in the inner parts of the bone. At that time point, there were no significant differences between all four kinds of implants. Some differences between four implant variants were revealed in bones of rabbits euthanized after 3 weeks after implant placement. Significant increase in the bone volume and trabecular number was observed in bone blocks with implants associated with liposomes containing miRNA inhibitors, as compared to other three types of implants. Analysis of bone blocks six weeks after implant placement revealed high bone volume and trabecular density in parts of the bone in contact with implants associated with liposomes containing miRNA inhibitors. Meanwhile, other implants had some visible remaining areas of bone resorption at that time point. Thus, due to significantly accelerated integration, perforated implants associated with liposomes containing miRNA inhibitors may be promising alternative to conventional dental implants.

### Literature:

1. Saresella M, La Rosa F, Piancone F, et al. The NLRP3 and NLRP1 inflammasomes are activated in Alzheimer's disease. Mol Neurodegener. 2016;11:23. Published 2016 Mar 3. doi:10.1186/s13024-016-0088-1
2. Pahl HL. Activators and target genes of Rel/NF-kappaB transcription factors. Oncogene. 1999;18(49):6853-6866.doi:10.1038/sj.onc.1203239
3. Liu T, Zhang L, Joo D, Sun SC. NF-κB signaling in inflammation. Signal Transduct Target Ther. 2017;2:17023-. doi:10.1038/sigtrans.2017.23
4. Agnihotri R, Gaur S. Rheumatoid arthritis in the elderly and its relationship with periodontitis: a review. Geriatr Gerontol Int 2014;14:8-22
5. Blair HA, Deeks ED. Infliximab Biosimilar (CT-P13; Infliximab-dyyb): A Review in Autoimmune Inflammatory Diseases. BioDrugs. 2016;30(5):469-480. doi:10.1007/s40259-016-0193-2
6. Jha A, Upton A, Dunlop WC, Akehurst R. The Budget Impact of Biosimilar Infliximab (Remsima®) for the Treatment of Autoimmune Diseases in Five European Countries. Adv Ther. 2015;32(8):742-756. doi:10.1007/s12325-015-0233-1
7. Alles J, Fehlmann T, Fischer U, Backes C, Galata V, Minet M, et al.,An estimate of the total number of true human miRNAs, Nucleic Acids Research, Volume 47, Issue 7, 23 April 2019, Pages 3353-3364, https://doi.org/10.1093/nar/gkz097
8. R. Dai, S. A. Ahmed. MicroRNA, a new paradigm for understanding immunoregulation, inflammation, and autoimmune diseases. Transl Res 2011; 157:163-179.
9. R. C. Page, P. I. Eke. Case definitions for use in population-based surveillance of periodontitis. J Periodontol. 2007;78:1387-99.
10. P. I. Eke, B.A. Dye, L. Wei, G. O. Thornton-Evans, and R.J. Genco. Prevalence of Periodontitis in Adults in the United States: 2009 and 2010. J DENT RES 2012 Oct;91(10):914-20
11. Singh P. Understanding peri-implantitis: a strategic review. J Oral Implantol. 2011;37(5):622-626.doi:10.1563/AAID-JOI-D-10-00134
12. Lee CT, Huang YW, Zhu L, Weltman R. Prevalences of peri-implantitis and peri-implant mucositis: systematic review and meta-analysis. J Dent. 2017;62:1-12. doi:10.1016/j.jdent.2017.04.011
13. Lozano C, Duroux-Richard I, Firat H, Schordan E, Apparailly F. MicroRNAs: Key Regulators to Understand Osteoclast Differentiation?. Front Immunol. 2019;10:375. Published 2019 Mar 7. doi:10.3389/fimmu.2019.00375
14. Bhaumik D, Scott G, Schokrpur S, Patil C, Campisi J, Benz C. Expression of microRNA-146 suppresses NF-κB activity with reduction of metastatic potential in breast cancer cells. Oncogene (2008) 27:5643-7. doi:10.1038/onc.2008.171
15. Zheng C, Shu Y, Luo Y, Luo J. The role of miR-146a in modulating TRAF6-induced inflammation during lupus nephritis. Eur Rev Med Pharmacol Sci (2017) 21:1041-8.
16. Li M, Wang J, Fang Y, Gong S, Li M, Wu M, et al. MicroRNA-146a promotes mycobacterial survival in macrophages through suppressing nitric oxide production. Sci Rep (2016) 6:23351. doi:10.1038/srep23351
17. Ammari M, Presumey J, Ponsolles C, Roussignol G, Roubert C, Escriou V, et al. Delivery of miR-146a to Ly6C high monocytes inhibits pathogenic bone erosion in inflammatory arthritis. Theranostics. (2018) 8:5972-85. doi: 10.7150/thno.29313
18. Germanier Y, Tosatti S, Broggini N, Textor M, Buser D. Enhanced bone apposition around biofunctionalized sandblasted and acid-etched titanium implant surfaces. A histomorphometric study in miniature pigs. Clin Oral Implants Res. 2006; 17(3):251-257. doi:10.111111/j. 1600-0501.2005.01222.x
19. Xu W-D, Lu M-M, Pan H-F, Ye D-Q. Association of MicroRNA-146a with autoimmune diseases. Inflammation (2012) 35(4):1525-9. doi:10.1007/s10753-012-9467-0
20. Fushimi S, Nohno T, Nagatsuka H, Katsuyama H. Involvement of miR-140-3p in Wnt3a and TGFβ3 signaling pathways during osteoblast differentiation in MC3T3-E1 cells. Genes Cells. 2018;23:517-27.
21. O'Leary L, Sevinç K, Papazoglou IM, Tildy B, Detillieux K, Halayko AJ, et al. Airway smooth muscle inflammation is regulated by microRNA-145 in COPD. FEBS Lett (2016) 590:1324-34. doi:10.1002/1873-3468.12168
22. Yang B, Kang X, Xing Y, Dou C, Kang F, Li J, et al. Effect of microRNA-145 on IL-1β-induced cartilage degradation in human chondrocytes. FEBS Lett (2014) 588:2344-52. doi:10.1016/j.febslet.2014.05.033
23. He M, Wu N, Leong MC, et al. miR-145 improves metabolic inflammatory disease through multiple pathways. J Mol Cell Biol. 2020;12(2):152-162. doi:10.1093/jmcb/mjz015
24. Wang W, Yang L, Zhang D, Gao C, Wu J, Zhu Y, et al. MicroRNA-218 negatively regulates osteoclastogenic differentiation by repressing the nuclear factor-KB signaling pathway and targeting tumor necrosis factor receptor 1. Cell Physiol Biochem. (2018) 48:339-47. doi: 10.1159
25. Krzeszinski JY, Wei W, Huynh H, et al. miR-34a blocks osteoporosis and bone metastasis by inhibiting osteoclastogenesis and Tgif2 [retracted in: Nature. 2020 Jun;582(7810):134]. Nature. 2014;512(7515):431-435. doi:10.1038/nature13375
26. R. A. Irwandi, A. Vacharaksa. The role of microRNA in periodontal tissue: A review of the literature. Arch Oral Biol. 2016 Dec;72:66-74
27. Dorhoi A, et al. MicroRNA-223 controls susceptibility to tuberculosis by regulating lung neutrophil recruitment. J. Clin. Invest. 2013;123:4836-4848. doi: 10.1172/JCI67604
28. Fordham, J., Guilfoyle, K., Naqvi, A. et al. MiR-142-3p is a RANKL-dependent inducer of cell death in osteoclasts. Sci Rep 6, 24980 (2016). https://doi.org/10.1038/srep24980
29. Cheng P, Chen C, He HB, et al. miR-148a regulates osteoclastogenesis by targeting V-maf musculoaponeurotic fibrosarcoma oncogene homolog B. J Bone Miner Res. 2013;28(5):1180-1190. doi:10.1002/jbmr.1845
30. Shao D, Wang C, Sun Y, Cui L. Effects of oral implants with miR-122-modified cell sheets on rat bone marrow mesenchymal stem cells. Mol Med Rep. 2018;17(1):1537-1544. doi: 10.3892/mmr.2017.8094
31. Wang Z, Wu G, Feng Z, et al. Microarc-oxidized titanium surfaces functionalized with microRNA-21-loaded chitosan/hyaluronic acid nanoparticles promote the osteogenic differentiation of human bone marrow mesenchymal stem cells. Int J Nanomedicine. 2015;10:6675-6687. Published 2015 Oct 27. doi:10.2147/IJN.S94689
32. Wu K, Song W, Zhao L, et al. MicroRNA functionalized microporous titanium oxide surface by lyophilization with enhanced osteogenic activity. ACS Appl Mater Interfaces. 2013;5(7):2733-2744. doi:10.1021/am400374c
33. Song W, Yang C, Svend Le DQ, Zhang Y, Kjems J. Calcium-MicroRNA Complex-Functionalized Nanotubular Implant Surface for Highly Efficient Transfection and Enhanced Osteogenesis of Mesenchymal Stem Cells. ACS Appl Mater Interfaces. 2018;10(9):7756-7764. doi: 10.1021/acsami.7b18289
34. Liu X, Tan N, Zhou Y, et al. Delivery of antagomiR204-conjugated gold nanoparticles from PLGA sheets and its implication in promoting osseointegration of titanium implant in type 2 diabetes mellitus. Int J Nanomedicine. 2017;12:7089-7101. Published 2017 Sep 26. doi:10.2147/IJN.S124584
35. Andersen, M. O., Nygaard, J. V., Burns, J. S., Raarup, M. K., Nyengaard, J. R., et al. (2010) siRNA nanoparticle functionalization of nanostructured scaffolds enables controlled multilineage differentiation of stem cells. Mol Ther; 18:2018-2027.

## Claims

1. A titanium dental implant **characterized in that** it is functionalized with a selected combination of miRNA inhibitors.

2. The titanium implant according to claim 1, wherein the implant is perforated to accommodate liposomes.

3. The titanium implant according to claims 1-2, wherein the liposome size is preferably between 25 and 35 nM.

4. The titanium implant according to claims 1-3, wherein the liposomes contain miRNA inhibitors.

5. The titanium implant according to claim 4, wherein the miRNA inhibitors are selected to inhibit expression of miR-140-3p, -145-5p, -146a-5p and -195-5p.
